# EUROPÄISCHE PATENTSCHRIFT

(11) **EP 1 178 763 B1**
(45) Veröffentlichungstag und Bekanntmachung des Hinweises auf die Patenterteilung: **10.12.2008**
(21) Anmeldenummer: 00929480.2
(22) Anmeldetag: 03.05.2000
(51) Int. Cl.: A61F 2/14

(54) **RETINA-IMPLANTAT UND VERFAHREN ZUR HERSTELLUNG EINES SOLCHEN**
RETINA IMPLANT AND METHOD FOR PRODUCING THE SAME
IMPLANT RETINIEN ET PROCEDE PERMETTANT DE LE PRODUIRE

(30) Priorität: 07.05.1999 DE 19921398; 06.07.1999 DE 19931083
(43) Veröffentlichungstag der Anmeldung: 13.02.2002
(73) Patentinhaber: Retina Implant AG, 72770 Reutlingen (DE)
(72) Erfinder: NISCH, Wilfried, D-72072 Tübingen (DE); STETT, Alfred, D-72770 Reutlingen (DE); SCHUBERT, Markus, D-78569 Stuttgart (DE); GRAF, Michael, D-71229 Leonberg (DE); GRAF, Heinz, Gerhard, D-71106 Magstadt (DE); HÄMMERLE, Hugo, D-72072 Tübingen (DE); ZRENNER, Eberhart, D-72076 Tübingen (DE); STELZLE, Martin, D-72766 Reutlingen (DE); SACHS, H., D-93053 Regensburg (DE)
(74) Vertreter: Witte, Alexander
(86) Internationale Anmeldenummer: PCT/EP2000/003962
(87) Internationale Veröffentlichungsnummer: WO 2000/067676

(56) Entgegenhaltungen:
- US-A- 2 760 483
- US-A- 5 147 284
- US-A- 5 935 155
- TRIEU H K ET AL: "FLEXIBLE SILICON STRUCTURES FOR A RETINA IMPLANT" IEEE WORKSHOP ON MICRO ELECTRO MECHANICAL SYSTEMS,US,NEW YORK, NY: IEEE, 25. Januar 1998 (1998-01-25), Seiten 515-519, XP000829215 ISBN: 0-7803-4413-8
- JESINGER R A ET AL: "FLEXIBLE ELECTRODE ARRAY FOR RETINAL STIMULATION" PROCEEDINGS OF THE ANNUAL INTERNATIONAL CONFERENCE OF THE ENGINEERING IN MEDICINE AND BIOLOGY SOCIETY,US,NEW YORK, IEEE, Bd. CONF. 14, 29. Oktober 1992 (1992-10-29), Seite 2393 XP000346990
- ROUSH W: "ENVISIONING AN ARTIFICIAL RETINA" SCIENCE,US,AMERICAN ASSOCIATION FOR THE ADVANCEMENT OF SCIENCE,, Bd. 268, Nr. 5211, 5. Mai 1995 (1995-05-05), Seiten 637-638, XP000541767 ISSN: 0036-8075

## Beschreibung

Die Erfindung betrifft ein Retina-Implantat mit einem Chip zur subretinalen Implantation, mit einer Empfänger-Spule zur induktiven Einkopplung von elektromagnetischer Energie, die mit einer Wandlereinrichtung zur Umwandlung einer in der Empfänger-Spule induzierten Wechselspannung in eine zur Stromversorgung des Chips geeignete Gleichspannung gekoppelt ist.

Die Erfindung betrifft ferner ein Verfahren zur Herstellung eines solchen Retina-Implantats.

Ein derartiges Implantat ist grundsätzlich bekannt.

In jüngster Zeit werden Retina-Implantate entwickelt, die zur Behandlung von Patienten vorgesehen sind, deren Sehvermögen vollständig oder teilweise durch Retina-bedingte Defekte verlorgengegangen ist. Im Prinzip soll hierzu ein lichtempfindlicher Chip in den subretinalen Raum unterhalb der Retina implantiert werden, der eine Vielzahl von Pixelelementen besitzt, über den ein auf die Retina durch die noch intakte Linse des Auges projiziertes Bild aufgenommen, in elektrische Signale umgesetzt werden soll und über eine Vielzahl von Stimulationselektroden in elektrische Stimuli für die an dem Chip anliegenden Zellen der Retina umgesetzt werden sollen, um so das Sehvermögen des erblindeten oder teilweise erblindeten Patienten wiederherzustellen bzw. zu verbessern.

Aus der US-A-4 628 933 ist ein Retina-implantat bekannt, das jedoch nicht zur subretinalen Implantation sondern zur epiretinalen Implantation bestimmt ist, d.h. der lichtempfindliche Chip soll nicht in den subretinalen Raum sondern unmittelbar auf die Oberfläche der Retina implantiert werden. Die Stimulationselektroden sind demzufolge nicht an der der Linse zugewandten Seite des Chips sondern an dessen Rückseite vorgesehen.

Es hat sich gezeigt, daß sowohl bei den subretinalen Implantaten als auch bei den epiretinalen Implantaten die Zufuhr von externer Energie notwendig ist, um die aufgenommenen Lichtsignale aktiv verstärken zu können und in Stimulationssignale für die anliegenden Zellen umsetzen zu können.

Hierzu wird gemäß der US-A-4 628 933 vorgeschlagen, mittels elektromagnetischer Induktion Energie auf das Implantat zu übertragen. Hierzu ist eine Empfänger-Spule um den Umfang des Implantat-Chips gewickelt, mittels derer HF-Energie, die von einer externen, beispielsweise an einem geeigneten Brillengestell befindlichen Energiequelle abgegeben wird, aufgenommen und in elektrische Energie zum Betrieb des Chips umgesetzt wird.

Da eine derartige Spule jedoch mit einer ausreichenden Windungszahl versehen sein muß, einen erheblichen Spulendurchmesser für die effektive Einkopplung der Hochfrequenzenergie aufweisen muß und geeignete Einrichtungen zur Gleichrichtung und Glättung der induzierten Wechselspannung vorgesehen sein müssen, wäre ein derartiger Chip zur Implantation in das menschliche Auge wegen seiner erheblichen Größe kaum geeignet.

Aus diesem Grunde wurden in jüngster Zeit subretinale Implantate entwickelt, die nicht über die HF-Einkopplung von elektromagnetischer Energie, sondern mittels unsichtbarer Infrarotstrahlung mit Energie versorgt werden, die von einer geeigneten photovoltaischen Schicht in elektrische Energie umgesetzt wird.

Ein derartiges subretinales Implantat ist beispielsweise aus der WO 98/17343 bekannt.

Als nachteilig wird hierbei jedoch angesehen, daß zum Betrieb des Implantats ständig Infrarotstrahlung in das Auge eingestrahlt werden muß.

Die Aufgabe der Erfindung besteht demnach darin, ein verbessertes Retina-Implantat anzugeben, das über induktive Einkopplung von elektromagnetischer Energie versorgt wird und das auf möglichst einfache Weise ohne die Gefahr von Komplikationen in den subretinalen Raum implantiert werden kann.

Ferner soll ein Verfahren zur Herstellung eines solchen Retina-Implantats angegeben werden.

Diese Aufgabe wird gelöst durch ein Retina-Implantat mit den Merkmalen des Anspruches 1.

Auf diese Weise wird es ermöglicht, eine ausreichend dimensionierte Empfänger-Spule zu verwenden, die außerhalb des Augapfels angeordnet wird und in vorteilhafter Weise durch ein Operationsverfahren eingesetzt werden kann, bei dem es vermieden wird, das Implantat durch den Glaskörper des Auges hindurchzuführen.

Da die Empfänger-Spule außerhalb des Auges auf der Sclera fixiert wird, kann der Chip, der in den subretinalen Raum implantiert werden soll, in geeigneter Weise dimensioniert werden, die zur Implantation in den subretinalen Raum besonders vorteilhaft ist. Dagegen kann die Empfänger-Spule mit einem ausreichend großen Querschnitt und einer ausreichend hohen Windungszahl versehen werden, ohne daß hiermit nachteilige Einflüsse verbunden sind.

In bevorzugter Weiterbildung der Erfindung sind die Empfänger-Spule, der Chip, sowie zum Chip führende Zuleitungen in einem flachen Kunststoffkörper aufgenommen.

Hierdurch wird eine kompakte Ausführung ermöglicht und eine komplikationsarme Implantation durch eine Sclerainzision an der Seite im Bereich des Äquators des Auges ermöglicht, durch die das Ende des flachen Kunststoffkörpers, in dem sich der Chip befindet, in den subretinalen Raum eingebracht werden kann. Das Operationsverfahren, das hierbei verwendet wird, ähnelt teilweise dem in der DE 197 41 487 A1 beschriebenen Operationsverfahren.

Gemäß einer bevorzugten Weiterbildung der Erfindung ist die Wandlereinrichtung als vom Chip getrennte Einheit ausgebildet, die zur Positionierung am Augapfel außerhalb der Sclera geeignet ist.

Durch diese Maßnahme wird der subretinal zu implantierende Teil des gesamten Retina-Implantats weiter verkleinert, da die Wandlereinrichtung Gleichrichter und Glättungskondensatoren aufweisen muß, die einen gewissen Platzbedarf besitzen und somit nicht unmittelbar mit dem Chip kombiniert werden können, ohne dessen Abmessungen merklich zu vergrößern.

Durch eine derartige Ausführung wird demnach der in den Augapfel subretinal einzuführende Teil des Implantats weiter verkleinert, so daß dieser Teil des Kunststoffkörpers als dünne Folie ausgebildet werden kann, in deren Ende der Chip integriert werden kann.

Der Kunststoffkörper weist in bevorzugter Weiterbildung der Erfindung einen ringförmigen Abschnitt mit einer zentralen Ausnehmung auf, an den sich ein flacher Fortsatz anschließt.

Auf diese Weise kann die Empfänger-Spule in besonders geeigneter Form in den ringförmigen Abschnitt des Kunststoffkörpers eingebracht werden, während der Chip am Ende des flachen Fortsatzes vorgesehen werden kann.

Gemäß einer weiteren bevorzugten Ausgestaltung der Erfindung ist der ringförmige Abschnitt zur Umschließung des lateralen Augenmuskelansatzes dimensioniert.

Diese Maßnahme hat den Vorteil, daß sich eine besonders günstige Anordnung und Positionierung der Empfänger-Spule auf dem Auge erreichen läßt. Bei der Implantation muß hierzu der Augenmuskel durchtrennt und anschließend wieder angenäht werden, was bei den heutigen Operationsmethoden jedoch keinerlei Problem darstellt.

Bei einer solchen Anordnung können die Erreger-Spulen zur Energieversorgung etwa seitlich im Bereich der Schläfen z.B. an einem Brillengestell angeordnet werden.

Die Wandlereinrichtung ist dagegen in bevorzugter Ausführung am Kunststoffkörper in einer an die Empfänger-Spule angrenzenden Position aufgenommen.

Dadurch wird die Wandlereinrichtung, die einen gewissen Platzbedarf aufweist, in besonders günstiger Weise positioniert.

Gemäß einer weiteren Ausführung der Erfindung weist die Empfänger-Spule in den ringförmigen Abschnitt integrierte, vorzugsweise spiralförmig angeordnete Windungen auf.

Auf diese Weise kann die Empfänger-Spule in vorteilhafter Weise in den ringförmigen Abschnitt des Runststoffkörpers integriert werden.

In zusätzlicher Weiterbildung dieser Ausführung ist der ringförmige Abschnitt in Umfangsrichtung in eine Mehrzahl von Abschnitten aufgeteilt, die durch Zwischenräume voneinander getrennt sind und elektrisch und mechanisch miteinander verbunden sind.

Durch diese Maßnahme wird die Flexibilität des ringförmigen Abschnitts verbessert, so daß sich der ringförmige Abschnitt des Kunststoffkörpers selbst dann, wenn dieser aus einem relativ zugsteifen Material besteht, gut an die gewölbte Oberfläche des Auges anlegen läßt.

Hierbei können die Abschnitte jeweils durch mindestens einen Steg in Radialrichtung miteinander verbunden sein oder aber die einzelnen Abschnitte Teile einer spiralförmig zusammenhängenden Spule sein.

Vorzugsweise weisen die einzelnen Abschnitte jedoch jeweils mehrere Windungen auf und sind durch Stege in Radialrichtung miteinander verbunden, über die auch die elektrische Verbindung zwischen benachbarten Windungen erfolgt.

Auf diese Weise läßt sich eine besonders gute Flexibilität und Anpassung an die gewölbte Oberfläche eines Augapfels erreichen, wobei der ringförmige Abschnitt zunächst als planarer Körper hergestellt werden kann, der erst anschließend in die gewünschte gewölbte Form gebracht wird.

Gemäß einer weiteren Abwandlung kann der ringförmige Abschnitt des Kunststoffkörpers auch eine mäanderförmige Form aufweisen, um so eine größere Flexibilität zu erreichen.

Gemäß einer weiteren Ausführung der Erfindung sind zumindest zwischen der Wandlereinrichtung und einer zum Chip oder zur Empfänger-Spule führenden Zuleitung Mittel zur Zugentlastung vorgesehen.

Die Zugentlastung kann beispielsweise durch eine mäanderförmige Ausführung etwa im Bereich zwischen dem ringförmigen Abschnitt und dem flachen Fortsatz im Bereich der Aufnahme der Wandlereinrichtung oder in einem sich an die Wandlereinrichtung unmittelbar anschließenden Bereich erreicht werden.

Auf diese Weise wird die elektrische Verbindung zwischen Empfänger-Spule, Wandlereinrichtung und Chip gegen nachteilige Einflüsse geschützt, die sich aus der ständigen Bewegung des Augapfels ergeben können, so daß auch noch Jahre nach einer Implantation eine zuverlässige Spannungsversorgung des implantierten Chips sichergestellt ist.

Die Empfänger-Spule weist in bevorzugter Weiterbildung der Erfindung eine Windungszahl von etwa 50 bis 200 Windungen, vorzugsweise von etwa 100 Windungen auf.

Mit einer derartigen Windungszahl läßt sich eine geeignete Energieübertragung und eine ausreichende Spannung an der Empfänger-Spule gewährleisten.

Eine derartige Empfänger-Spule kann etwa in Kombination mit einer externen Sender-Spule mit einer Frequenz von 1 MHz betrieben werden, die etwa 1000 Windungen bei einem Durchmesser von etwa 50 mm aufweist und z.B. an einem Brillengestell befestigt werden kann.

In bevorzugter Weiterbildung der Erfindung weist die Empfänger-Spule einen Außendurchmesser von etwa 12 bis 20 mm und einen Innendurchmesser an der Ausnehmung von etwa 8 bis 16 mm auf.

Mit einer derartigen Dimensionierung läßt sich eine vorteilhafte Anpassung an die anatomischen Gegebenheiten des menschlichen Auges gewährleisten.

Gemäß einer weiteren bevorzugten Ausführung der Erfindung ist der flache Fortsatz mit einer Mehrzahl von Perforationen versehen.

Diese können beispielsweise in einem Rastermaß von ca. 0,1 bis 1 mm angeordnet werden und einen Durchmesser im Bereich zwischen etwa 20 und 200 µm aufweisen.

Hierdurch wird eine Diffusion von Nährstoffen und Sauerstoff zwischen Pigmentepithel und Retina ermöglicht, so daß nachteilige durch die Implantation eines undurchlässigen Teils bedingte Folgen vermieden werden.

Der Chip ist gemäß einer weiteren bevorzugten Ausgestaltung der Erfindung in dem flachen Fortsatz eingebettet, wobei seine aktive Seite zur Zellstimulation freiliegend an der Oberfläche angeordnet ist.

Hierdurch ist sichergestellt, daß der Chip mit einem geeigneten Operationsverfahren durch eine Sclerainzision im Bereich des lateralen Augenmuskels von der Seite her in dem subretinalen Raum vorgeschoben werden kann.

Gemäß einer weiteren bevorzugten Ausgestaltung der Erfindung weist der flache Kunststoffkörper Laschen zur Befestigung am Augapfel auf.

Auf diese Weise wird ein sicherer Sitz in der gewünschten Position am Augapfel gewährleistet. Die Laschen können hierbei sowohl am ringförmigen Abschnitt als auch am flachen Fortsatz, vorzugsweise im Bereich der Aufnahme der Wandlereinrichtung, vorgesehen sein.

Gemäß einer weiteren Ausgestaltung der Erfindung weist die Wandlereinrichtung Mittel zur Signalverarbeitung eines mit elektromagnetischer Energie übertragenen Informationssignals, insbesondere zur Aufbereitung eines Referenzsignals für die Umgebungshelligkeit auf.

Auf diese Weise wird die ohnehin zur Energieankopplung notwendige elektromagnetische Energie gleichzeitig zur Übertragung von Informationssignalen genutzt, beispielsweise zur Übertragung eines Referenzsignals für die Umgebungshelligkeit, um eine Anpassung der Amplitude der Ausgangssignale des Chips bei stark veränderlicher Umgebungshelligkeit zu gewährleisten. Auf diese Weise kann die Leistungsfähigkeit des implantierten Chips deutlich verbessert werden.

In bevorzugter Weiterbildung der Erfindung wird der flache Kunststoffkörper auf einem Hilfssubstrat erzeugt, von dem er anschließend wieder abgelöst wird.

Auf diese Weise wird das Herstellungsverfahren deutlich vereinfacht.

Hierbei werden die Leiterbahnen vorzugsweise auf der Oberfläche durch Dünnschichtverfahren (Aufdampfen oder Sputtern) erzeugt und anschließend photolithographisch mikrostrukturiert.

Auf diese Weise kann durch die Verwendung bewährter Herstellverfahren eine hohe Präzision und Qualität gewährleistet werden.

Die Aufgabe der Erfindung wird ferner durch ein Verfahren zur Herstellung des neuen Retina-Implantats gelöst, mit einem Chip zur subretinalen Implantation, mit einer Empfänger-Spule zur induktiven Einkopplung von elektromagnetischer Energie, und mit einer Wandlereinrichtung zur Umwandlung einer in die Empfänger-Spule induzierten Wechselspannung in eine zur Stromversorgung des Chips geeignete Gleichspannung, das folgende Schritte aufweist:
- Herstellen eines flachen Kunststoffkörpers mit einem ringförmigen Abschnitt mit einer zentralen Ausnehmung, an den sich ein flacher Fortsatz anschließt,
- Aufbringen von metallischen Leiterbahnen in spiralförmiger Anordnung auf eine Oberfläche des ringförmigen Abschnitts des Kunststoffkörpers, um die Windungen der Empfänger-Spule zu erzeugen und um elektrische Verbindungsleitungen zwischen der Empfängerspule, der Wandlereinrichtung und dem Chip zu schaffen,
- Befestigen des Chips und der Wandlereinrichtung am Kunststoffkörper,
- Aufbringen einer Isolationsschicht über den metallischen Leiterbahnen.

Auf diese Weise läßt sich erfindungsgemäß ein Retina-Implantat herstellen, das einen dünnen folienartigen Fortsatz mit dem zu implantierenden Chip aufweist, die als eine zusammenhängende flache Einheit ausgebildet ist, auf besonders günstige Weise mit minimalen Komplikationen implantiert werden kann und außerdem eine hohe Beständigkeit über eine ausreichend lange Zeit aufweist.

Gemäß einer Weiterbildung der Erfindung wird die Wandlereinrichtung am Kunststoffkörper durch Kleben, durch Bondieren mit den metallischen Leiterbahnen und/oder durch Umspritzen mit einer Kunststoffmasse befestigt.

Auf diese Weise läßt sich eine dauerhafte und Beständige Befestigung der Wandlereinrichtung am Kunststoffkörper erreichen und eine gute Abdichtung nach außen.

Gemäß weiteren Merkmalen der Erfindung werden der Kunststoffkörper und vorzugsweise auch die Isolationsschicht aus Polyimid, Polymethylmetacrylat (PMMA) oder Epoxydharz hergestellt. Gleichermaßen können diese Materialien zum Einbetten des Chips und/oder zum Umspritzen der Wandlereinrichtung verwendet werden.

Diese Materialien haben sich als besonders biologisch verträglich und beständig erwiesen, wobei Polyimid oder PMMA besonders bevorzugt sind.

Der Kunststoffkörper einschließlich der Isolationsschicht wird vorzugsweise mit einer Dicke von etwa 5 bis 15 Mikrometer, vorzugsweise von etwa 10 Mikrometer, erzeugt.

Auf diese Weise lassen sich sowohl die Empfänger-Spule als auch der der Chip und die notwendigen Zuleitungen in einem dünnen folienartigen Körper unterbringen, der gut zur Implantation geeignet ist.

Hierbei weisen die Leiterbahnen vorzugsweise eine Stärke von etwa 0,5 bis 2 Mikrometer, vorzugsweise von etwa 1 Mikrometer auf.

Eine solche Dimensionierung hat sich als ausreichend für eine zuverlässige elektrische Verbindung erwiesen.

Die Leiterbahnen werden vorzugsweise durch Aufdampfen bzw. Sputtern erzeugt, was eine günstige Herstellung erlaubt. Sie bestehen vorzugsweise aus Gold, Titan, Platin, Iridium, Aluminium oder Kupfer.

Es versteht sich, daß die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung bevorzugter Ausführungsbeispiele unter Bezugnahme auf die Zeichnung. Es zeigen:
- Fig. 1: in perspektivischer Ansicht ein herauspräpariertes Auge mit eingeführtem Retina-Implantat;
- Fig. 2: ein Ansicht des Retina-Implantats in vergrößertem Maßstab in leicht abgewandelter Ausführung;
- Fig. 3: einen Schnitt durch den ringförmigen Bereich des Implantats längs der Linie III-III in vergrößerter Darstellung;
- Fig. 4: den ringförmigen Bereich gemäß Fig. 3 in gekrümmter Form unter Biegebeanspruchung;
- Fig. 5: einen Ausschnitt des ringförmigen Bereichs des Implantats in gegenüber Fig. 3 nochmals vergrößerte Darstellung und
- Fig. 6: einen schematisierten Querschnitt durch ein Auge mit eingeführtem Implantat.

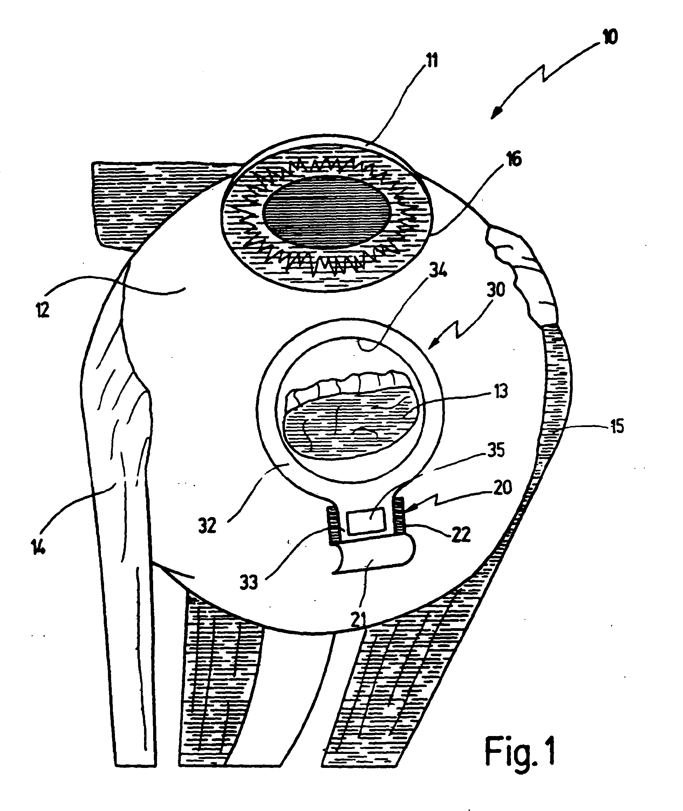

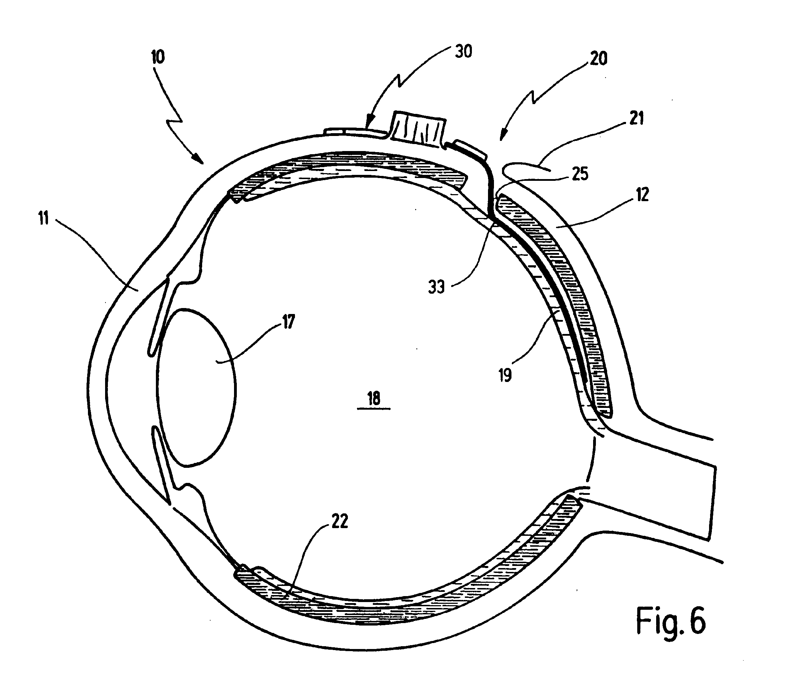

In den Figuren 1 und 6 ist ein Augapfel, in den ein erfindungsgemäßes Implantat eingeführt ist, insgesamt mit 10 bezeichnet. Der Augapfel 10 weist an seiner Vorderseite eine Hornhaut (Cornea) 11 und im übrigen eine Lederhaut (Sclera) 12 auf. Der Limbus corneae, d.h. der Rand der Hornhaut 11, ist in Fig. 1 mit 16 bezeichnet. Ferner sind in Fig. 1 die seitlichen Augenmuskeln 14, 15 erkennbar.

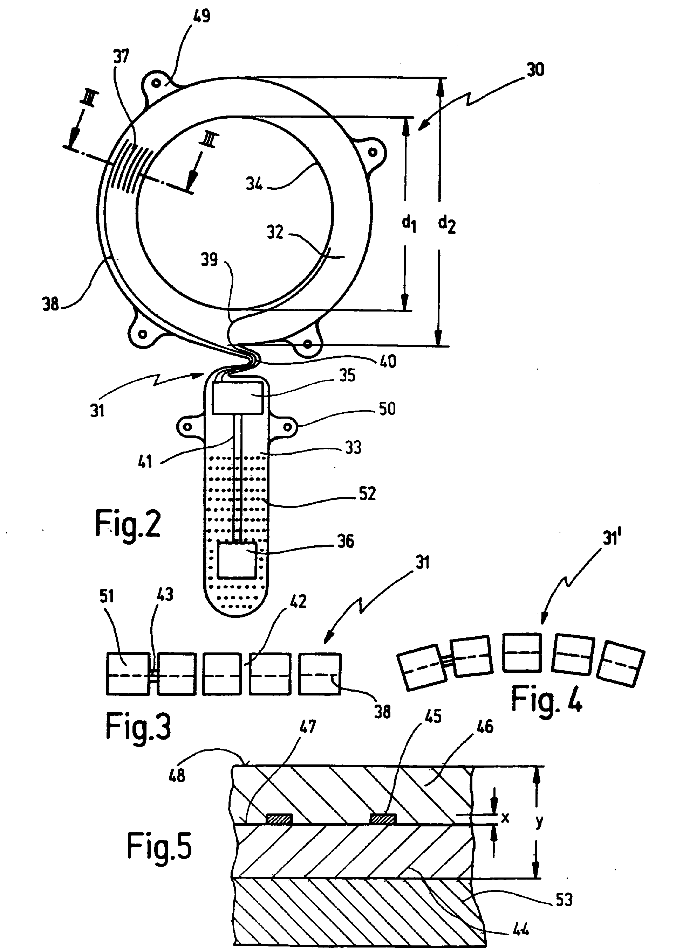

Vorne am Augapfel 10 wurde der laterale Augenmuskel 13 mittels eine Schnittes durchgetrennt. Ein erfindungsgemäßes Retina-Implantat, das insgesamt mit 30 bezeichnet ist, besitzt einen ringförmigen Abschnitt 32 und einen sich daran anschließenden flachen Fortsatz 33 an dessen äußeren Ende, wie aus Fig. 2 näher zu ersehen ist, ein Chip 36 zur subretinalen Implantation vorgesehen ist. In Fig. 1 ist erkennbar, wie das Retina-Implantat mit seinem ringförmigen Abschnitt 32 um den lateralen Augenmuskel 13 herumgelegt wurde und mit seinem flachen Fortsatz 33 durch eine Sclerainzision 20 in den subretinalen Raum eingeschoben wurde.

Aufbau und Herstellung des Retina-Implantats wird nachfolgend anhand der Fig. 2 bis 5 näher beschrieben.

In Fig. 2 ist das Retina-Implantat in der Aufsicht vergrößert dargestellt. Hierbei ist in Abwandlung zu der Ausführung gemäß Fig. 1 noch eine Zugentlastung vorgesehen, wie nachfolgend noch erläutert wird.

Das Retina-Implantat 30 umfaßt eine Empfänger-Spule 37 zur Aufnahme von extern eingestrahlter elektromagnetischer Energie (HF-Energie), eine Wandlereinrichtung 35, um die in der Empfänger-Spule 37 induzierte Wechselspannung gleichzurichten und zu glätten und ggf. auf eine voreingestellte Spannung zu stabilisieren, sowie den Chip 36 zur subretinalen Implantation.

Die Wandlereinrichtung 35, die wegen der notwendigen Glättungskondensatoren einen gewissen Platzbedarf hat, ist als vom Chip 36 räumlich getrennte Einheit ausgebildet, die an den ringförmigen Bereich 32 angrenzend am Beginn des flachen Fortsatzes 33 angeordnet ist. Die Wandlereinrichtung 35 besteht z.B. aus Gleichrichtern, Glättungskondensatoren bzw. wiederaufladbaren Dünnfilmbatterien und einem Spannungsstabilisator. Zusätzlich kann sie Komponenten zum Dekodieren und zur Verarbeitung von mit der elektromagnetischen Energie übertragenen Informationssignalen, etwa von Referenzsignalen für die Umgebungshelligkeit, aufweisen.

Das Retina-Implantat weist einen flachen, vorzugsweise aus Polyimid bestehenden Kunststoffkörper 31 auf, in dem die verschiedenen Komponenten aufgenommen sind, bzw. an dem diese Komponenten befestigt sind. Im ringförmigen Bereich 32 des Kunststoffkörpers 31 ist eine zentrale Ausnehmung 34 mit einem Durchmesser d₁ von etwa 8 bis 16 mm vorgsehen. Der Außendurchmesser d₂ des ringförmigen Abschnitts beträgt etwa 12 bis 20 mm. Im ringförmigen Abschnitt 32 ist die Empfänger-Spule 37 aufgenommen, deren Windungen beispielhaft mit 38 in Fig. 2 angedeutet sind. Die Enden der Empfänger-Spule 37 sind über eine Zugentlastung 40, die wie aus Fig. 2 ersichtlich aus einer zickzackförmigen oder mäanderförmig gewundenen Zunge bestehen kann, mit der Wandlereinrichtung 35 verbunden. Die Wandlereinrichtung 35 ist über Verbindungsleitungen 41 mit dem Chip 36 verbunden, der am äußeren Ende des flachen Fortsatzes 33 in den Kunststoffkörper 31 eingebettet ist, wobei seine aktive Oberfläche, die zur Stimulation von Zellen dient, natürlich freiliegt. Um eine Befestigung des Implantats 30 am Augapfel 10 in seiner endgültigen Position zu erleichtern, sind am ringförmigen Abschnitt 32 und am flachen Fortsatz 33 Laschen 49 bzw. 50 vorgesehen. Wie aus Fig. 2 ferner ersichtlich ist, weist der flache Fortsatz 33 in seinem Bereich, der zum späteren Einschieben in den subretinalen Raum bestimmt ist, eine Vielzahl von Perforationen 52 auf, die beispielsweise in einem Rastermaß von etwa 0,1 bis 1 mm angeordnete sein können und einen Durchmesser etwa im Bereich zwischen 20 und 200 µm aufweisen können. Auf diese Weise wird eine Diffusion von Nährstoffen und Sauerstoff zwischen Pigmentepithel und Retina ermöglicht, um so nachteilige Einflüsse des aus isolierendem Material bestehenden Implantats zu vermeiden.

Der ringförmige Abschnitt 33, in dem die Empfänger-Spule 37 ausgebildet ist, ist vorzugsweise, wie aus den Figuren 3 und 4 ersichtlich, in Umfangsrichtung in eine Mehrzahl von koaxial zueinander angeordneten ringförmigen Abschnitten 51 aufgeteilt, die durch Zwischenräume 42 voneinander getrennt sind und die durch Radialstege 43 mechanisch und elektrisch miteinander verbunden sind.

Jeder dieser Abschnitte 51 enthält eine Mehrzahl von Windungen 38 und besitzt z.B. eine Breite in der Größenordnung von etwa 0,5 mm, während die Gesamtbreite etwa im Bereich zwischen 3 und 4 mm liegt.

In Fig. 3 ist beispielhaft ein einziger Radialsteg 43 zwischen den beiden äußeren Abschnitten 51 erkennbar, über den die beiden benachbarten Abschnitte 51 mechanisch zusammenhängen und über den auch die elektrische Verbindung zwischen den benachbarten Windungen 38 erfolgt. Die Verbindung zu den weiter innen liegenden Abschnitten 51 erfolgt gleichfalls über Radialstege 43, die vorzugsweise winkelmäßig versetzt an anderen Stellen des ringförmigen Abschnittes angeordnet sind, um so eine maximale Flexibilität der Empfängerspule 37 zu erreichen, da der Kunststoffkörper 31, wie nachfolgend noch erläutert wird, als ebener Körper erzeugt wird, sich jedoch das Kunststoffmaterial möglichst ohne Wellen und Falten an die Oberfläche des gewölbten Augapfels anlegen soll. Zusätzlich ist es möglich, diese isolierten Abschnitte 51 nach der vollständigen Herstellung des Kunststoffkörpers in eine sphärische Form zu bringen, die der Wölbung des Augapfels angepaßt ist und zum Beispiel durch Vergießen mit Silikon in dieser gewölbten Form zu fixieren, so daß die Handhabung bei der späteren Implantation vereinfacht wird.

Das Retina-Implantat kann beispielsweise folgendermaßen hergestellt werden:

Zunächst wird eine dünne Kunststoffschicht 44 gemäß Fig. 5, die z.B. aus Polyimid, PMMA oder Epoxydharz bestehen kann, auf ein planares Hilfssubstrat 53 wie z.B. einen Glas-, Silizium- oder Keramikwafer laminiert bzw. aufgespinnt. Bei dem Hilfssubstrat 53 kann es sich beispielsweise auch um eine Metallfolie handeln.

Nach der Erzeugung dieser dünnen Kunststoffschicht oder Folie 44 auf der Oberfläche des Hilfssubstrats 53 werden metallische Leiterbahnen 45 auf der Oberfläche der Folie 44 erzeugt. Hierzu wird das Metall, z.B. Gold, Titan, Platin, Iridium, Aluminium oder Kupfer auf die Oberfläche aufgedampft bzw. gesputtert. Hieraus werden dann sämtliche metallischen Leiterbahnen 45, also die Windungen 38 der Empfänger-Spule 37, Verbindungen zwischen den einzelnen Abschnitten 51 der Empfänger-Spule 37, zur Wandlereinrichtung 35 über die Zugentlastung 40 führende Anschlußleitungen sowie Verbindungsleitungen 41 von der Wandlereinrichtung 35 zum Chip 36 durch eine anschließende photolithographische Mikrostrukturierung erzeugt. Eine vom inneren Ende der Empfängerspule 37 nach außen verlaufende Brücke 39 muß in der Regel gesondert angeschlossen werden. Der Chip 36 und die Wandlereinrichtung 35 werden mit der Oberfläche 47 der Folie 44 z.B. durch leitfähiges Kleben oder Bonden befestigt und eine Isolationsschicht zur vollständigen Isolation der Leiterbahnen 45 aufgebracht, die aus dem gleichen Kunststoff wie der zunächst auf das Hilfssubstrat 53 aufgebrachte Kunststoff, also etwa aus Polyimid oder PMMA, bestehen kann.

Der so gebildete Körper wird anschließend wieder von dem Hilfssubstrat 53 abgelöst. Je nach den geometrischen Verhältnissen werden Chip 36 und die Wandlereinrichtung 35 auch erst nach Aufbringen der Isolationsschicht 46 auf die Folie 44 am Kunststoffkörper 31 durch leitfähiges Kleben oder Bonden befestigt.

Auch die Zwischenräume 42 zwischen benachbarten Abschnitten 51 der Empfängerspule 37 und die mäanderförmig ausgebildete Zugentlastung 40 können im Rahmen der photolithographischen Mikrostrukturierung erzeugt werden.

Dagegen werden die Perforationen 52 im flachen Fortsatz 33 vorzugsweise anschließend zum Beispiel durch Laserbohren erzeugt.

Die einzelnen Abschnitte 51 der Empfängerspule 37 können zusätzlich zum Beispiel mittels Silikon in eine sphärische Form vergossen werden, die der Wölbung eines Augapfels angepaßt ist.

Die metallischen Leiterbahnen weisen eine Stärke x von etwa 0,5 bis 2 Mikrometer, vorzugsweise von etwa 1 Mikrometer auf.

Nach dem Aufbringen der Isolationsschicht 46 weist die Empfänger-Spule 37 zusammen mit der Folie 44 eine Gesamtdicke y von etwa 10 Mikrometer auf.

Das Retina-Implantat 30 kann etwa auf folgende Weise implantiert werden:

Nach Eröffnung der Bindehaut am Rande der Hornhaut 11 am schläfenwärtigen Teil des Augapfels 10 wird ein Zügelfaden angelegt und das Auge nach innen gerollt. Die Bindehaut wird anschließend zur Seite geschoben, ggf. als Lappen. Am Ansatz des geraden lateralen Augenmuskels 13 wird ein Haltefaden in den Muskelansatz eingebracht und der Muskel dann durchtrennt und samt Haltefaden zur Seite geklappt. Etwas oberhalb oder, wie in Fig. 1 gezeigt, etwas unterhalb des Augenmuskels 13 wird dann in die Sclera etwas unterhalb des lateralen Augenmuskels 13 eine Sclerainzision 20 eingebracht und ein kleiner viereckiger Lappen 21 fensterflügelförmig aufgeschnitten und nach unten aufgeklappt.

Durch die beiden Ecken des Lappens 21 werden Haltefäden gelegt und dann der Lappen nach hinten weggeklappt, wie in Fig. 1 zu sehen. Unter Einsatz von blutstillenden Mitteln werden die unter der Sclera 12 liegende Aderhaut 22 und Pigmentepithel schlitzförmig eröffnet (vgl. Fig. 6), ohne die Retina 19 selbst zu perforieren. Ggf. unter Sicht mit einer Faseroptik wird das Ende des den Chip 36 tragenden Fortsatzes 33 in den Aderhautschlitz eingeschoben und bis in den subretinalen Raum vorgeschoben, bis sich der Fortsatz zu etwa 4/5 innerhalb des Auges 10 befindet und das Ende mit dem Chip 36 die endgültige Position erreicht hat. Ggf. kann vorher mit einem flexiblen, dem Fortsatz des Retina-Implantats 30 ähnelnden Plastikspatel der Kanal dadurch freigelegt werden, daß die Retina 19 etwas vom Pigmentepithel abgetrennt wird.

Hat das Ende, das den Chip 36 trägt, seine endgültige Position im Bereich der Macula erreicht, wird sie in dieser Position an der Sclera außen dadurch befestigt, daß sie mit zwei Kunststoff-Fäden, die durch kleine Perforationslöcher an den Laschen 50 am Fortsatz 33 gezogen werden, mit Einzelknoten an der Sclera befestigt werden. Der ringförmige Abschnitt 32 mit der Empfänger-Spule 37 wird in ähnlicher Weise mit zwei weiteren Einzelknopfnähten, die durch Perforationen an den Laschen 49 an der dem Fortsatz 33 gegenüberliegende Seite der Empfänger-Spule 37 gezogen werden, befestigt. Die Empfänger-Spule 37 wird so gelegt, daß sich die augapfelseitige Wurzel des lateralen geraden Augenmuskels 13 innerhalb der Ausnehmung 34 des ringförmigen Abschnitts 32 befindet.

Anschließend wird der fensterartige Lappen 21 der Sclera 11 an seinen beiden Ecken mit zwei weiteren Einzelknopfnähten geschlossen. Danach wird der laterale gerade Augenmuskel 13 mit Hilfe des Haltefadens wieder nach vorne gezogen und über den ringfömigen Breich 32 hinweg an seiner ursprünglichen Wurzel mit Muskelnähten befestigt. Dann wird mit einigen wenigen Einzelknopfnähten die Bindehaut wieder angeheftet und der Zügelfaden entfernt. Nachdem die Position des Chips und die Verhältnisse am Augengrund mit Hilfe der direkten und indirekten Ophtalmosopie geprüft worden sind, wird zuletzt ein Salbenverband aufgelegt.

## Patentansprüche

1. Retina-Implantat mit
- einem zur Implantation im Inneren eines Augapfels (10) in Kontakt mit der Retina vorgesehenen Chip (36), und
- einer Empfänger-Spule (37) zur induktiven Einkopplung von elektromagnetischer Energie, die mit einer Wandlereinrichtung (35) zur Umwandlung einer in die Empfänger-Spule (37) induzierten Wechselspannung in eine zur Stromversorgung des Chips (36) dienende Gleichspannung gekoppelt ist,
**dadurch gekennzeichnet, dass** der Chip (36) in den Subretinalraum des Auges implantierbar ist, dass die Empfänger-Spule (37) als vom Chip (36) getrenntes Bauelement und zur Positionierung außerhalb der Sclera (12) auf dem Augapfel (10) ausgebildet ist, und dass der Chip (36) mit der Empfänger-Spule (37) über eine Zuleitung (41) verbunden ist, die im implantierten Zustand das Innere mit dem Äußeren des Augapfels (10) verbindet.

2. Retina-Implantat nach Anspruch 1, **dadurch gekennzeichnet, dass** die Empfänger-Spule (37), der Chip (36), sowie zum Chip (36) führende Zuleitungen (41) in einem flachen Kunststoffkörper (31) aufgenommen sind.

3. Retina-Implantat nach Anspruch 1 oder 2, **dadurch gekennzeichnet, dass** die Wandlereinrichtung (35) als vom Chip (36) getrennte Einheit ausgebildet ist.

4. Retina-Implantat nach Anspruch 2 oder 3, **dadurch gekennzeichnet, dass** der Kunststoffkörper (31) einen ringförmigen Abschnitt (32) mit einer zentralen Ausnehmung (34) aufweist, an den sich ein flacher Fortsatz (33) anschließt.

5. Retina-Implantat nach Anspruch 4, **dadurch gekennzeichnet, dass** der ringförmige Abschnitt (32) zur Umschließung des lateralen geraden Augenmuskelansatzes (13) dimensioniert ist.

6. Retina-Implantat nach Anspruch 5, **dadurch gekennzeichnet, dass** die Wandlereinrichtung (35) am Kunststoffkörper (31) in einer an die Empfänger-Spule (37) angrenzenden Position aufgenommen ist.

7. Retina-Implantat nach einem der Ansprüche 3 bis 6, **dadurch gekennzeichnet, dass** die Empfänger-Spule (37) in den ringförmigen Abschnitt (32) integrierte, vorzugsweise spiralförmig angeordnete Windungen (38) aufweist.

8. Retina-Implantat nach einem der Ansprüche 4 bis 7, **dadurch gekennzeichnet, dass** der ringförmige Abschnitt (32) in Umfangsrichtung in eine Mehrzahl von Abschnitten (51) aufgeteilt ist, die durch Zwischenräume (42) voneinander getrennt sind und elektrisch und mechanisch miteinander verbunden sind.

9. Retina-Implantat nach Anspruch 8, **dadurch gekennzeichnet, dass** die Abschnitte (51) jeweils durch mindestens einen Steg (51) in Radialrichtung miteinander verbunden sind.

10. Retina-Implantat nach Anspruch 8, **dadurch gekennzeichnet, dass** die Abschnitte spiralförmig zusammenhängen.

11. Retina-Implantat nach einem der Ansprüche 8 bis 10, **dadurch gekennzeichnet, dass** die Abschnitte (51) zu einem wölbungsmäßig an die Oberfläche eines Augapfels (10) angepassten annähernd sphärischen Körper vergossen sind.

12. Retina-Implantat nach einem der vorhergehenden Ansprüche, **dadurch gekennzeichnet, dass** zumindest zwischen der Wandlereinrichtung (35) und einer zum Chip (36) oder zur Empfänger-Spule (37) führenden Zuleitung (41) Mittel zur Zugentlastung (40) vorgesehen sind.

13. Retina-Implantat nach einem der vorhergehenden Ansprüche, **dadurch gekennzeichnet, dass** die Empfänger-Spule (37) eine Windungszahl von etwa 50 bis 200 Windungen, vorzugsweise von etwa 100 Windungen aufweist.

14. Retina-Implantat nach einem der vorhergehenden Ansprüche, **dadurch gekennzeichnet, dass** die Empfänger-Spule (37) einen Außendurchmesser (d₂) von etwa 12 bis 20 mm und einen Innendurchmesser (d₁) an der Ausnehmung (34) von etwa 8 bis 16 mm aufweist.

15. Retina-Implantat nach einem der vorhergehenden Ansprüche, **dadurch gekennzeichnet, dass** der flache Fortsatz (33) mit einer Mehrzahl von Perforationen (52) versehen ist.

16. Retina-Implantat nach einem der vorhergehenden Ansprüche, **dadurch gekennzeichnet, dass** der Chip (36) in dem flachen Fortsatz (33) eingebettet ist.

17. Retina-Implantat nach einem der vorhergehenden Ansprüche, **dadurch gekennzeichnet, dass** der flache Kunststoffkörper (31) Laschen (49, 50) zur Befestigung am Augapfel (10) aufweist.

18. Retina-Implantat nach einem der vorhergehenden Ansprüche, **dadurch gekennzeichnet, dass** die Wandlereinrichtung (35) Mittel zur Signalverarbeitung eines mit der elektromagnetischen Energie übertragenen Informationssignals, insbesondere zur Aufbereitung eines Referenzsignals für die Umgebungshelligkeit, aufweist.

19. Verfahren zur Herstellung eines Retina-Implantats (30) gemäß einem der Ansprüche 1 bis 18,
- Herstellen eines flachen Kunststoffkörpers (31) mit einem ringförmigen Abschnitt (32) mit einer zentralen Ausnehmung (34), an den sich ein flacher Fortsatz (33) anschließt,
- Aufbringen von metallischen Leiterbahnen (45) in spiralförmiger Anordnung auf eine Oberfläche (47) des ringförmigen Abschnitts (32) des Kunststoffkörpers (31), um die Windungen (38) der Empfänger-Spule (37) zu erzeugen und um elektrische Verbindungsleitungen (41) zwischen der Empfänger-Spule (37), der Wandlereinrichtung (35) und dem Chip (36) zu schaffen,
- Befestigen des Chips (36) und der Wandlereinheit (35) am Kunststoffkörper (31),
- Aufbringen einer Isolationsschicht (46) über den metallischen Leiterbahnen (45).

20. Verfahren nach Anspruch 19, bei dem der flache Kunststoffkörper (31) auf einem Hilfssubstrat (53) erzeugt wird, von dem er anschließend wieder abgelöst wird.

21. Verfahren nach Anspruch 20, bei dem die Leiterbahnen (45) auf der Oberfläche (47) durch ein Dünnschichtverfahren erzeugt werden und anschließend photolithographisch mikrostrukturiert werden.

22. Verfahren nach Anspruch 19, 20 oder 21, bei dem die Wandlereinrichtung (35) am Kunststoffkörper (31) durch Kleben, durch Bondieren mit den metallischen Leiterbahnen (45) und/oder durch Umspritzen mit einer Kunststoffmasse befestigt wird.

23. Verfahren nach einem der Ansprüche 19 bis 22, bei dem der ringförmige Abschnitt (32) in Umfangsrichtung in eine Mehrzahl von Abschnitten (51) aufgeteilt wird, die durch Zwischenräume (42) voneinander getrennt sind.

24. Verfahren nach Anspruch 23, bei dem die Abschnitte (51) zu einem wölbungsmäßig an die Oberfläche eines Augapfels (10) angepassten annähernd kugelkalottenförmigen Körper vergossen werden.

25. Verfahren nach einem der Ansprüche 19 bis 24, bei dem der Kunststoffkörper (31) aus Polyimid, Polymethylmethacrylat (PMMA) oder Epoxyharz hergestellt wird.

26. Verfahren nach einem der Ansprüche 19 bis 25, bei dem der Kunststoffkörper (31) einschließlich der Isolationsschicht (46) mit einer Dicke von etwa 5 bis 15 Mikrometer, vorzugsweise von etwa 10 Mikrometer erzeugt wird.

27. Verfahren nach einem der Ansprüche 19 bis 26, bei dem die metallischen Leiterbahnen (45) mit einer Stärke (X) von etwa 0,5 bis 2 Mikrometer, vorzugsweise von etwa 1 Mikrometer erzeugt werden.

28. Verfahren nach einem der Ansprüche 19 bis 27, bei dem der flache Kunststoffkörper (31) im Bereich seines flachen Fortsatzes (33) mit Perforationen (52) versehen wird.

## Claims

1. A retina implant, having
- a chip (36) for implantation into an eyeball (10) in contact with the retina, and
- a receiver coil (37) for conductively coupling thereinto electromagnetic energy, the coil being connected to converter means (35) for converting an alternating voltage induced into the receiver coil (37) into a direct voltage suited for supplying the chip (36),
**characterized in that**
- the chip (36) is designed such as to be implantable into the subretinal space of the eye, that the receiver coil (37) is configured as a unit separate from the chip (36) and is adapted to be positioned on the eyeball (10) outside the sclera (12), and that the chip (36) is connected to the receiver coil (37) via connecting leads (41) which, in the implanted state, connects the inside of the eyeball (10) with its outside.

2. The retina implant of claim 1, **characterized in that** the receiver coil (37), the chip (36) as well as connecting leads (41) leading to the chip (36) are housed within a flat plastic material body (31).

3. The retina implant of claim 1 or 2, **characterized in that** the converting means (35) is configured as a unit separate from the chip (36).

4. The retina implant of claim 2 or 3, **characterized in that** the plastic material body (31) comprises an annular portion (32) having a central opening (34), the annular portion (32) being adjoined by a flat extension (33).

5. The retina implant of claim 4, **characterized in that** the annular portion (32) is dimensioned as to enclose the lateral straight eye muscle rudiment.

6. The retina implant of claim 5, **characterized in that** the converting means (35) is comprised at the plastic material body (31) in a position adjacent the receiver coil (37).

7. The retina implant of any one of claims 3 to 6, **characterized in that** the receiver coil (37) comprises windings (38) integrated into the annular portion (32) and being, preferably, configured spiralled.

8. The retina implant of any one of claims 4 to 7, **characterized in that** the annular portion (32) is subdivided into a plurality of sections (51) in a circumferential direction, the sections (51) being separated from each other by gaps (42) and being electrically and mechanically interconnected.

9. The retina implant of claim 8, **characterized in that** the sections (51) are interconnected with each other by at least each one fin (43) in a radial direction.

10. The retina implant of claim 8, **characterized in that** the sections adhere to each other in a spiralled configuration.

11. The retina implant of any one of claims 8 to 10, **characterized in that** the sections (51) are molded together in a roughly spherical body having a curvature being adapted to the surface of an eyeball (10).

12. The retina implant of any one of the preceding claims, **characterized in that** strain relief means (40) are provided at least between the converter means (35) and a connection lead (41) leading to the receiver coil (37) or to the chip (36).

13. The retina implant of any one of the preceding claims, **characterized in that** the receiver coil (37) has a number of windings of about between 50 and 200, preferably about 100.

14. The retina implant of any one of the preceding claims, **characterized in that** the receiver coil (37) has an outer diameter (d₂) of about between 12 and 20 mm and an inner diameter (d₁) at the opening (34) of about between 8 and 16 mm.

15. The retina implant of any one of the preceding claims, **characterized in that** the flat extension (33) is provided with a plurality of perforations (52).

16. The retina implant of any one of the preceding claims, **characterized in that** the chip (36) is embedded in the flat extension (33).

17. The retina implant of any one of the preceding claims, **characterized in that** the flat plastic material body (31) is provided with lugs (49, 50) for affixing the plastic material body (31) to the eye ball (10).

18. The retina implant of any one of the preceding claims, **characterized in that** the converter means (35) comprises means for processing an information signal transmitted together with the electromagnetic energy, in particular for processing a reference signal indicative for ambient brightness.

19. A method for manufacturing a retina implant (30) according to any one of claims 1 to 18, comprising the steps of:
- manufacturing a flat plastic material body (31) having an annular portion (32) with a central opening (34), the annular portion (32) being adjoined by a flat extension (33),
- depositing metallic strip conductors (45) on a surface (47) of the annular portion (32) in a spiralled configuration for making windings (38) of the receiver coil (37) and for creating connecting leads (41) between the receiver coil (37), the converter means (35) and the chip (36),
- affixing the chip (36) and the converter means (35) to the plastic material body (31),
- depositing an isolation layer (46) over the metallic strip conductors (45).

20. The method of claim 19, wherein the flat plastic material body (31) is generated on an auxiliary substrate (53) and is then separated therefrom.

21. The method of claim 20, wherein the strip conductors (45) are generated on the surface (47) by means of a thin film process and are then microstructured photolithographically.

22. The method of claims 19, 20 or 21, wherein the converter means (35) is affixed to the plastic material body (31) by gluing, bonding with the metallic strip conductors (45) and/or by injection-molding in a plastic material mass.

23. The method of any one of claims 19 to 22, wherein the annular portion (32) is subdivided into a plurality of sections (51) in a circumferential direction, the sections (51) being separated from each other by gaps (42).

24. The method of claim 23, wherein the sections (51) are molded together in a roughly spherical calotte body having a curvature being adapted to the surface of an eyeball (10).

25. The method of any one of claims 19 to 24, wherein the plastic material body (31) is manufactured from polyimide, polymethacrylate (PMMA) or epoxy resin.

26. The method of any one of claims 19 to 25, wherein the plastic material body (31) together with the isolation layer (46) is manufactured with a thickness of about between 5 and 15 micrometers, preferably about 10 micrometers.

27. The method of any one of claims 19 to 26, wherein the metallic strip conductors (45) are generated with a thickness (X) of about between 0.5 to 2 micrometers, preferably about 1 micrometer.

28. The method of any one of claims 19 to 27, wherein the flat plastic material body (31) is provided with perforations (52) in the area of the flat extension (33).

## Revendications

1. Implant de rétine ayant
- une puce (36) prévue pour l'implantation à l'intérieur d'un globe oculaire (10) en contact avec la rétine, et
- une bobine réceptrice (37) destinée au couplage inductif d'énergie électromagnétique, qui est couplée à un dispositif de transducteur (35) pour convertir une tension alternative induite dans la bobine réceptrice (37) en une tension continue servant à l'alimentation en courant de la puce (36),
**caractérisé en ce que** la puce (36) peut être implantée dans l'espace sous-rétinal de l'oeil, **en ce que** la bobine réceptrice (37) est réalisée en tant qu'élément de construction séparé de la puce (36) et en vue du positionnement à l'extérieur de la sclérotique (12) sur le globe oculaire (10), et **en ce que** la puce (36) est reliée à la bobine réceptrice (37) par le biais d'un conduit (41), qui relie dans l'état implanté l'intérieur à l'extérieur du globe oculaire (10).

2. Implant de rétine selon la revendication 1, **caractérisé en ce que** la bobine réceptrice (37), la puce (36) ainsi que les conduits (41) conduisant à la puce (36) sont logés dans un corps plat en matière synthétique (31).

3. Implant de rétine selon la revendication 1 ou 2, **caractérisé en ce que** le dispositif de transducteur (35) est réalisé sous la forme d'une unité séparée de la puce (36).

4. Implant de rétine selon la revendication 2 ou 3, **caractérisé en ce que** le corps en matière synthétique (31) inclut une section de forme annulaire (32) ayant un évidemment central (34) auquel s'adjoint un prolongement plat (33).

5. Implant de rétine selon la revendication 4, **caractérisé en ce que** la section sous forme annulaire (32) est dimensionnée pour envelopper l'amorce du muscle de l'oeil droit latéral (13).

6. Implant de rétine selon la revendication 5, **caractérisé en ce que** le dispositif de transducteur (35) est disposé dans une position adjacente à la bobine réceptrice (37) sur le corps en matière synthétique (31).

7. Implant de rétine selon l'une quelconque des revendications 3 à 6, **caractérisé en ce que** la bobine réceptrice (37) présente, dans la section sous forme annulaire (32), des enroulements intégrés, arrangés, de préférence, sous forme de spirale (38).

8. Implant de rétine selon l'une quelconque des revendications 4 à 7, **caractérisé en ce que** la section sous forme annulaire (32) est subdivisée en direction du pourtour en une pluralité de sections (51) qui sont séparées les unes des autres par des interstices (42) et qui sont reliées les unes aux autres du point de vue électrique et mécanique.

9. Implant de rétine selon la revendication 8, **caractérisé en ce que** les sections (51) sont reliées à chaque fois les unes aux autres en direction radiale par au moins une barrette (51).

10. Implant de rétine selon la revendication 8, **caractérisé en ce que** les sections sont liées ensemble sous forme de spirale.

11. Implant de rétine selon l'une quelconque des revendications 8 à 10, **caractérisé en ce que** les sections (51) sont coulées pour former un corps approximativement sphérique, adapté en termes de courbure à la surface d'un globe oculaire (10).

12. Implant de rétine selon l'une quelconque des revendications précédentes, **caractérisé en ce que** l'on prévoit, au moins entre le dispositif de transducteur (35) et un conduit (41) conduisant à la puce (36) ou à la bobine réceptrice (37), des moyens en vue de la décharge de traction (40).

13. Implant de rétine selon l'une quelconque des revendications précédentes, **caractérisé en ce que** la bobine réceptrice (37) présente un nombre d'enroulements d'environ 50 à 200 enroulements, de préférence, d'environ 100 enroulements.

14. Implant de rétine selon l'une quelconque des revendications précédentes, **caractérisé en ce que** la bobine réceptrice (37) présente un diamètre externe (d₂) d'environ 12 à 20 mm et un diamètre interne (d₁) dans l'évidemment (34) d'environ 8 à 16 mm.

15. Implant de rétine selon l'une quelconque des revendications précédentes, **caractérisé en ce que** le prolongement plat (33) est pourvu d'une pluralité de perforations (52).

16. Implant de rétine selon l'une quelconque des revendications précédentes, **caractérisé en ce que** la puce (36) est noyée dans le prolongement plat (33).

17. Implant de rétine selon l'une quelconque des revendications précédentes, **caractérisé en ce que** le corps en matière synthétique plat (31) présente des attaches (49, 50) en vue de la fixation au globe oculaire (10).

18. Implant de rétine selon l'une quelconque des revendications précédentes, **caractérisé en ce que** le dispositif de transducteur (35) présente des moyens en vue du traitement de signaux d'un signal d'information transmis à l'aide d'énergie électromagnétique, en particulier en vue de l'apprêt d'un signal de référence pour la luminosité de l'environnement.

19. Procédé en vue de la fabrication d'un implant de rétine (30) selon l'une quelconque des revendications 1 à 18, qui présente les étapes suivantes:
- la fabrication d'un corps en matière synthétique plat (31) ayant une section sous forme annulaire (32) avec un évidemment central (34), auquel s'adjoint un prolongement plat (33),
- l'application de pistes conductrices métalliques (45) en disposition sous forme de spirale sur une surface (47) de la section sous forme annulaire (32) du corps en matière synthétique (31), pour produire les enroulements (38) de la bobine réceptrice (37) et pour réaliser les conduits de liaison électriques (41) entre la bobine réceptrice (37), le dispositif de transducteur (35), et la puce (36),
- la fixation de la puce (36) et de l'unité de transducteur au corps en matière synthétique (31),
- l'application d'une couche d'isolation (46) sur les pistes conductrices métalliques (45).

20. Procédé selon la revendication 19, lors duquel le corps en matière synthétique plat (31) est produit sur un substrat auxiliaire (53), duquel il est ensuite à nouveau détaché.

21. Procédé selon la revendication 20, lors duquel les pistes conductrices (45) sont produites par un procédé à couches minces sur la surface (47) et subissent ensuite une micro structuration par voie photolithographique.

22. Procédé selon la revendication 19, 20 ou 21, lors duquel le dispositif de transducteur (35) est fixé sur le corps en matière synthétique (31) par collage, par liaison aux pistes conductrices métalliques et/ou par pulvérisation à l'aide d'une masse de matière synthétique.

23. Procédé selon l'une quelconque des revendications 19 à 22, lors duquel la section sous forme annulaire (32) est subdivisée en direction du pourtour en une pluralité de sections (51) qui sont séparées les unes des autres par des interstices (42).

24. Procédé selon la revendication 23, lors duquel les sections (51) sont coulées pour former un corps approximativement en forme de calotte sphérique, adapté du point de vue courbure à la surface d'un globe oculaire (10).

25. Procédé selon l'une quelconque des revendications 19 à 24, lors duquel le corps en matière synthétique plat (31) est fabriqué à partir de polyamide, de polyméthacrylate de méthyle (PMMA) ou de résine époxyde.

26. Procédé selon l'une quelconque des revendications 19 à 25, lors duquel le corps en matière synthétique plat (31), y compris la couche d'isolation (46), est produit à raison d'une épaisseur d'environ 5 à 15 micromètres, de préférence, d'environ 10 micromètres.

27. Procédé selon l'une quelconque des revendications 19 à 26, lors duquel les pistes conductrices métalliques (45) sont produites dans une taille (X) d'environ 0,5 à 2 micromètres, de préférence, d'environ 1 micromètre.

28. Procédé selon l'une quelconque des revendications 19 à 27, lors duquel le corps en matière synthétique plat (31) est pourvu de perforations (52) dans le domaine de son prolongement plat (33).
